# EUROPÄISCHE PATENTSCHRIFT

(11) **EP 0 490 428 B1**
(45) Veröffentlichungstag und Bekanntmachung des Hinweises auf die Patenterteilung: **13.03.1996**
(21) Anmeldenummer: 91203155.6
(22) Anmeldetag: 03.12.1991
(51) Int. Cl.: A61B 5/103, G01J 3/28, G01N 21/47

(54) **Vielkanalspektrometer**
Spectrometer with multichannel detection
Spectromètre à détection multicanale

(30) Priorität: 07.12.1990 DE 4039070
(43) Veröffentlichungstag der Anmeldung: 17.06.1992
(73) Patentinhaber: Philips Patentverwaltung GmbH, D-22335 Hamburg (DE); Philips Electronics N.V., NL-5621 BA Eindhoven (NL)
(72) Erfinder: Martens, Gerhard, Dr., W-2359 Henstedt-Ulzburg 2 (DE)
(74) Vertreter: Hartmann, Heinrich, Dipl.-Ing.

(56) Entgegenhaltungen:
- DE-A- 2 726 606
- DE-A- 2 758 141
- US-A- 3 874 799
- US-A- 4 170 987
- PHYSICS IN MEDECINE AND BIOLOGY. Bd. 34, Nr. 7, Juli 1989, LONDON GB Seiten 807 - 820; J.W. FEATHER ET AL: 'A portable scanning reflectance spectrophotometer using visible wavelengths for the rapid measurement of skin pigments'

## Beschreibung

Die Erfindung betrifft ein Vielkanalspektrometer zur Untersuchung von Pigmentmalen mit einer Auswerte- und Anzeigeeinrichtung und optischen Mitteln zur Bestimmung und zur Abbildung von Remissionsspektren einer Hautoberfläche auf einer auswertbaren Bildebene unter Berücksichtigung von Weiß-Standards.

Messungen von Körperfarben gewinnen in vielen Bereichen der Technik zunehmend an Bedeutung. Im medizinischen Bereich ist man daran interessiert, objektive Maßzahlen für Körperfarben zu gewinnen, um z.B. diagnostische Informationen über die Hautoberfläche zu gewinnen. Bei herkömmlichen Farbmeßgeräten wird das Licht, das von der Hautoberfläche eines Körperpunktes abgestrahlt wird, mit Hilfe eines Monochromators in sein Spektrum zerlegt und anschließend zur Bestimmung der Farbkoordinaten mit vorgegebenen Normspektralwertkurven verrechnet. Bei nicht selbstleuchtenden Objekten muß dabei von einer bestimmten vorgegebenen Beleuchtungsart ausgegangen oder das Primärlichtspektrum miterfaßt und das Remissionsspektrum des Körperpunktes darauf korrigiert werden.

Ein derartiges Spektrometer ist aus der DE-OS 27 26 606 bekannt. Dabei handelt es sich im wesentlichen um ein sogenanntes medizinisches Spektralfotometer mit einem Distanzring am objektivseitigen Ende zur Farbmessung mit Hilfe der Spektrometrie von reflektiertem Licht, also der Messung der Remission (Reflektivität) einer z.B. Hautoberfläche, bei der auch noch als Referenz ein Weiß-Standard alternierend benutzt wird. Ein solches Spektrometer ermöglicht die Ermittlung des Remissionsspektrums von einem Punkt der Oberfläche.

Bei strukturierten Oberflächen, z.B. einer Hautoberfläche mit Pigmentmalen oder der Oberfläche eines Pigmentmals selbst, ist es notwendig, nicht nur einen definierten Punkt bezüglich seines Remissionsspektrums zu vermessen, sondern diese Informationen von einer Vielzahl von Hautoberflächenpunkten zu bestimmen. Im Idealfall ist es wünschenswert, von jedem Punkt der Hautoberfläche das Remissionsspektrum zu bestimmen. Eine aufwendige optische Punkt für Punkt-Abtastung der Oberfläche ist dazu notwendig. Bei der Untersuchung von u.U. malignen Pigmentmalen ist ferner eine berührungsfreie Untersuchung wünschenswert.

Aus der DE-OS 38 15 743 ist eine Vorrichtung zur Messung und Auswertung von Eigenfluoreszenzspektren organischer Gewebeflächen bekannt. Bei dieser Vorrichtung wird vorgeschlagen, zur Erfassung der spektralen Verteilung von Fluoreszenzlicht der Hautoberfläche dieselbe spaltenförmig durch Projektion eines Spaltbildes auf die Hautoberfläche zu beleuchten. Dies hat zur Folge, daß für alle Punkte des von der Lichtquelle auf die Hautoberfläche projizierten Spaltbildes ein gemeinsames über alle Punkte gemitteltes Spektrum erfaßt wird. Eine, wie oben bereits ausgeführt wurde, wünschenswerte optische Punkt für Punkt-Abtastung der Hautoberfläche ist mit dieser Vorrichtung nicht möglich.

Aus der US-A-38 74 799 ist ein Reflektometer zur Bestimmung der Farbkoordinaten einer Probenoberfläche bekannt.

Bei dieser Anordnung erfolgt die spektrale Vermessung der Probe und zweier Referenzproben zeitlich nacheinander. Das Spektrum wird als Mittelwert über die gesamte Probenoberfläche vermessen. Eine optische Punkt für Punkt-Abtastung ist hiermit ebenfalls nicht möglich.

Aus der US-A-41 70 987 ist eine Vorrichtung zur Messung und Auswertung des von einer Probenoberfläche reflektierten Lichts bekannt. Mit Hilfe eines Spiegels wird die Probenoberfläche punktweise abgetastet, und das davon reflektierte Licht wird mit Hilfe von Farbfiltern in drei spektrale Komponenten zerlegt, aus denen pixelweise Bilder für Diagnosezwecke erstellt werden. Ein Weiß-Standard wird hierbei nicht berücksichtigt.

Aufgabe der vorliegenden Erfindung ist es, ein optisches Vielkanalspektrometer zu schaffen, zur berührungsfreien Ermittlung der unterschiedlichen Remissionsspektren der Punkte mindestens einer Zeile zu untersuchenden Hautoberfläche unter kontinuierlicher Berücksichtigung einer Primärlichtkorrektur.

Erfindungsgemäß wird diese Aufgabe durch die im kennzeichnenden Teil des Anspruchs 1 angegebenen Merkmale gelöst.

Die Spaltblende läßt nur einen zeilenförmigen Bildausschnitt passieren. Ein nachgeordnetes System von zwei Abbildungslinsen, zwischen denen das Beugungsgitter angeordnet ist, erzeugt ein zeilenförmiges Zwischenbild und bildet es auf der Bildebene ab. Die Gitter des Beugungsgitters sind parallel zur Richtung des Spaltes der Spaltblende ausgerichtet.

Gemäß einer besonderen Ausgestaltung der Erfindung besteht der Weiß-Standard aus beispielsweise gepreßtem Bariumsulfat und kann im Bereich der beobachteten Zeile der Hautoberfläche in einem kleinen Abschnitt auf dem Pigmentmal selbst oder daneben auf der Hautoberfläche befestigt, z. B. aufgeklebt sein.

Zur Untersuchung der ganzen Fläche des Pigmentmals auf der Hautoberfläche kann vorteilhaft vorgesehen sein, daß das erfindungsgemäße Vielkanalspektrometer orthogonal zur Spaltrichtung und zur optischen Achse des Linsensystems verschiebbar und justierbar angeordnet ist, so daß erfindungsgemäße Remissionsspektren bestimmbar von quasi jeder Zeile des Pigmentmals erzeugt werden können.

Gemäß einer besonderen Ausgestaltung kann das erfindungsgemäße Vielkanalspektrometer ein Gehäuse haben und anstelle eines Objektivs vor einer Restlichtkamera montiert werden.

Die Mittel zur optisch scharfen Abbildung werden vorteilhaft durch eine Linearverschiebungseinheit der Objektivlinse gebildet. Es kann auch vorgesehen sein, daß die Objektivlinse in ein Zoomobjektiv mit variabler Brennweite integriert ist.

Die erfindungsgemäße Anordnung des Linsensystems, der Spaltblende und des Beugungsgitters hat zur Folge, daß durch Beugung des parallelen Lichtstrahlbündels am Beugungsgitter in der Bildebene in einer Richtung senkrecht zur Richtung des Spaltes die Spektren der einzelnen Spaltbildpunkte entstehen und sich ebenfalls senkrecht zum Spaltbild und zweimal vom direkten Spaltbild nullter Ordnung ausgehend spiegelbildlich zueinander in zwei entgegengesetzten Richtungen erstrecken. Die Korrektur, d.h. die Normierung der Remissionssprektren des z.B. Pigmentmals bezüglich der spektralen Verteilung des Primärlichtes geschieht über das Remissionsspektrum des Weiß-Standards, das gleichzeitig miterfaßt wird, weshalb praktisch jedes Primärlicht verwendet werden kann, solange die gerade betrachtete Zeile beleuchtet ist.

Der in der Bildebene angeordnete zweidimensionale Detektor dient zur Detektion der Spektren der einzelnen Spaltbildpunkte der Hautoberfläche und gleichzeitig des Weiß-Standards, um letzlich die Farbkoordinaten für jeden Punkt der jeweils betrachteten Zeile über eine nachgeordnete Auswerte- und Anzeigevorrichtung zu berechnen und anzuzeigen. Die Berechnung erfolgt für jeden Punkt der Zeile aus den normierten Remissionsspektren und tabellierten Normspektralwertkurven, die in einem Speicher der Auswertevorrichtung abgelegt sein können.

Mit Hilfe des erfindungsgemäßen Vielkanalspektrometers kann somit berührungslos ein zeilenförmiger Ausschnitt aus der Hautoberfläche z.B. eines Pigmentmals für jeden Punkt simultan spektrometriert werden. Zur Abtastung der vollständigen Fläche bedarf es lediglich der entsprechenden mehrmaligen Verschiebung des Spektrometers. Häufig ist es jedoch zu diagnostischen Zwecken bereits ausreichend, wenn das Farbprofil beispielsweise einer Zeile bekannt ist.

Weitere vorteilhafte Ausgestaltungen der Erfindung ergeben sich aus den abhängigen Ansprüchen.

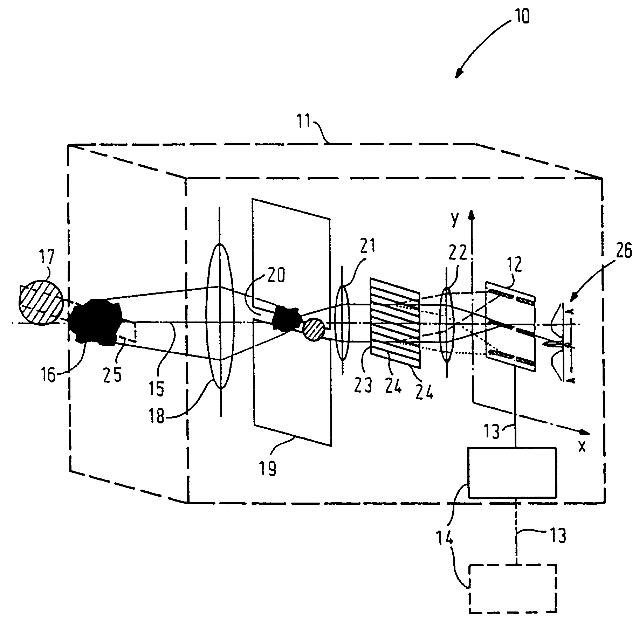

Ein Ausführungsbeispiel der Erfindung ist in der Zeichnung in der einzigen Fig. 1 dargestellt und wird im folgenden näher beschrieben.

Fig. 1 zeigt den prinzipiellen Aufbau und die Funktionsweise des erfindungsgemäßen Vielkanalspektrometers 10, das in einem Gehäuse 11 angeordnet ist und einen zweidimensionalen Detektor 12 enthält, der über eine Verbindungsleitung 13 mit einer beispielsweise außerhalb (gestrichelte Linie) des Gehäuses 11 angeordneten Auswerte- und Anzeigeeinrichtung 14 verbunden ist. Die Auswerte- und Anzeigeeinrichtung 14 (ausgezogene Linie) kann ebenfalls im Gehäuse 11 angeordnet sein.

Die optische Achse 15 des Vielkanalspektrometers 10 ist auf eine Hautoberfläche außerhalb des Gehäuses 11 gerichtet und besonders auf ein auf der Hautoberfläche befindliches Pigmentmal 16. Neben dem Pigmentmal 16 ist auf der nicht dargestellten Hautoberfläche eine beispielsweise in Tablettenform ausgeführte Probe aus gepreßtem Bariumsulfat als Weiß-Standard 17 befestigt. Es kann vorgesehen sein, daß der Weiß-Standard 17 auch auf bzw. teilweise auf dem Pigmentmal 16 angeordnet ist, dieses jedoch nicht wesentlich abdeckt. Mit einer nicht dargestellten Lichtquelle wird das Pigmentmal 16 und auch der Weiß-Standard 17 auf der Hautoberfläche spektral breitbandig beleuchtet. Es ist jedoch auch möglich, eine spektral schmalbandige Beleuchtung vorzusehen. Die Ausleuchtung des jeweils ausgewerteten Abschnitts bzw. der ausgewerteten Zeile 25 muß über den Abschnitt hinweg gleichmäßig sein.

Mit Hilfe einer im Gehäuse 11 befindlichen und z.B. verschiebbaren Objektivlinse 18 werden das Pigmentmal 16 und der Weiß-Standard 17 gleichzeitig scharf in den Spaltbereich einer Spaltblende 19 abgebildet. Der Abstand der Objektivlinse 18 vom Spalt 20 kann dazu über eine nicht dargestellte Linearverschiebungseinheit in Richtung der optischen Achse 15 variabel sein. Auch kann ein Zoomobjekt als Objektivlinse 18 vorgesehen sein. Vorteilhaft sind dann unterschiedliche Vergrößerungen einstellbar, ohne daß der Abstand zwischen Spektrometer und Hautoberfläche geändert werden muß. Die Spaltblende 19 weist den in der Zeichnungsebene horizontal verlaufenden Spalt 20 auf, der nur einen zeilenförmigen Ausschnitt 25 der Hautoberfläche, d.h. des Pigmentmals 16 und des Weiß-Standards 17 passieren läßt.

Über ein der Spaltblende 19 nachgeordnetes System von zwei Linsen 21 und 22 wird das zeilenförmige Zwischenbild in eine auswertbare Bildebene x, y projiziert. In dieser Bildebene x, y, die dem Doppellinsensystem aus den Linsen 21 und 22 nachgeordnet ist, ist der entsprechend empfindliche zweidimensionale optische Detektor 12 angeordnet. Zwischen den Linsen 21 und 22 ist ein Beugungsgitter 23 angeordnet, dessen Gitter 24 parallel zur Richtung des Spaltes 20 der Spaltblende 19 verlaufen. Das Doppellinsensystem aus den Linsen 21 und 22 ist derart zwischen der Spaltblende 19 und dem Detektor 12 angeordnet, daß der vorderseitige Brennpunkt der Linse 21 in der Spaltebene 19 und der rückseitige Brennpunkt der Linse 22 in der Bildebene x, y liegt. Die Parallelität des Lichtbündels im Doppellinsensystem wird also durch den Abstand zwischen der Linse 21 und der Spaltblende 19 besimmt. D.h. es ist notwendig, daß der dem Spalt 20 zugewandte Brennpunkt von Linse 21 genau in der Ebene des Spalts 20 liegt. Für den Detektor 12 und Linse 22 gilt, daß der detektorseitige Brennpunkt der Linse 22 genau in der Bildebene x, y, also des Detektors 12 liegt.

Die Beugung des parallelen Lichtstrahlbündels des Zwischenbilds erfolgt am Beugungsgitter 23 in einer Richtung orthogonal zur Richtung des Spaltes 20 und zur optischen Achse 15. Dies hat zur Folge, daß in der Bildebene x, y auf dem zweidimensionalen Detektor 12 die Spektren der einzelnen Spaltbildpunkte sich ebenfalls orthogonal zum Spaltbild erstrecken und zwar zweimal vom direkten Spaltbild nullter Ordnung ausgehend spiegelbildlich zueinander in zwei entgegengesetzten Richtungen. Dieser Sachverhalt ist jeweils schraffiert in der Fig. 1 auf dem Detektor 12 und in einem Diagramm 26 angedeutet. Letzteres ist zum besseren Verständnis lagerichtig neben dem Detektor 12 dargestellt.

Die Korrektur, d.h. Normierung der Remissionsspektren des Pigmentmals 16 bezüglich der spektralen Verteilung des Primärlichts geschieht über das Remissionsspektrum des Weiß-Standards 17, welches gleichzeitig miterfaßt wird, wie dies auch auf dem Detektor 12 schraffiert dargestellt ist. Die Berechnung der Farbkoordinaten wird in der Auswerteeinrichtung der Auswerte- und Anzeigeeinrichtung 14 für jeden Punkt der gerade betrachteten Zeile 25 des Pigmentmals 16 vorgenommen aus den normierten Remissionsspektren und tabellierten Normspektralwertkurven, die in einem Speicher der Auswerteeinrichtung gespeichert sind. Die Auswerteeinrichtung kann ein Mikroprozessor sein.

Wie bereits erwähnt, kann vorteilhaft das Gehäuse 11 ähnlich einem Kameraobjektiv ausgebildet sein, wodurch das Vielkanalspektrometer anstelle eines Objektivs vor einer Restlichtkamera montierbar ist, mit deren Hilfe dann die Spektren detektiert und an die Auswerte- und Anzeigeeinrichtung 14 weitergegeben werden können.

Eine Vorrichtung zum Bewegen, also Anheben bzw. Senken des Vielkanalspektrometers, um mehrere Zeilen 15 eines Pigmentmals auszuwerten, ist in Fig. 1 nicht dargestellt. Beispielsweise kann ein normales Kamerastativ eingesetzt werden.

Die in der vorstehenden Beschreibung, in der Zeichnung sowie in den Ansprüchen offenbarten Merkmale der Erfindung können sowohl einzeln als auch in beliebiger Kombination für die Verwirklichung der Erfindung in ihren verschiedenen Ausführungsformen wesentlich sein.

## Patentansprüche

1. Vielkanalspektrometer zur Untersuchung von Pigmentmalen, mit einer Auswerte- und Anzeigeeinrichtung und optischen Mitteln zur Bestimmung und zur Abbildung von Remissionsspektren einer Hautoberfläche auf einer auswertbaren Bildebene unter Berücksichtigung eines Weiß-Standards,
dadurch gekennzeichnet, daß die optischen Mittel ein auf die Hautoberfläche und den Weiß-Standard (17) gerichtetes Linsensystem (18, 21, 22) und eine Spaltblende (19) mit einem nachgeordneten, entsprechend einem Spalt (20) der Spaltblende (19) ausgerichteten Beugungsgitter (23) umfassen, daß das Beugungsgitter (23) zwischen zwei Abbildungslinsen (21, 22) des Linsensystems (18, 21, 22) geschaltet ist, zur gleichzeitigen Erzeugung des Remissionsspektrums der Punkte einer bestimmbaren Zeile (25) der Hautoberfläche und des Weiß-Standards (17) auf der dem Beugungsgitter (23) nachgeordneten, mit der Auswerte- und Anzeigeeinrichtung (14) verbundenen und durch einen zweidimensionalen Detektor gebildeten Bildebene (x, y).

2. Spektrometer nach Anspruch 1,
dadurch gekennzeichnet, daß das Linsensystem (18, 21, 22) auf der optischen Achse (15) der Abbildungslinsen (21, 22) eine Objektivlinse (18) mit Mitteln zur scharfen Abbildung der Hautoberfläche und des Weiß-Standards (17) auf der Spaltblende (19) enthält.

3. Spektrometer nach Anspruch 2,
dadurch gekennzeichnet, daß der Weiß-Standard (17) aus einer mindestens in der gerade ausgewerteten Zeile (25) der Hautoberfläche befestigten, z.B. verklebten Tablette aus beispielsweise gepreßtem Bariumsulfat besteht.

4. Spektrometer nach Anspruch 3,
dadurch gekennzeichnet, daß die Tablette neben einem Pigmentmal (16) auf der Hautoberfläche oder auf einem kleineren Abschnitt des Pigmentmals (16) befestigt ist und zusammen mit dem Pigmentmal (16) von der Objektivlinse (18) abgebildet wird.

5. Spektrometer nach Anspruch 4,
dadurch gekennzeichnet, daß das Spektrometer (10) orthogonal zur Spaltrichtung (20) und zur optischen Achse (15) verschiebbar und justierbar ist, zur Erzeugung von Remissionsspektren von mehreren Zeilen (25) der zu untersuchenden Hautoberfläche.

6. Sprektrometer nach Anspruch 5,
dadurch gekennzeichnet, daß das Spektrometer (10) ein Gehäuse (11) aufweist, mit welchem es anstelle eines Objektivs vor einer Restlichtkamera montierbar ist.

7. Spektrometer nach Anspruch 6,
dadurch gekennzeichnet, daß die Mittel zur optisch scharfen Abbildung durch eine Linearverschiebungseinheit der Objektivlinse (18) gebildet werden, oder die Objektivlinse (18) in einem Zoomobjektiv mit variabler Brennweite integriert ist.

8. Spektrometer nach Anspruch 7,
dadurch gekennzeichnet, daß dem Detektor (12) die Auswerte- und Anzeigeeinrichtung (14) zugeordnet ist, die die Berechnung und Anzeige der Farbkoordinaten für jeden Punkt der jeweils ausgewerteten Zeile (25) durchgeführt aus dem normierten Remissionsspektrum und tabellierten Normspektralwertkurven, die z.B. in der Auswerteeinrichtung gespeichert sind.

9. Spektrometer nach einem oder mehreren der vorhergehenden Ansprüche,
dadurch gekennzeichnet, daß mindestens die jeweils zur Auswertung gelangende Zeile (25) der Hautoberfläche und des Weiß-Standards (17) mit Licht beleuchtet ist.

## Claims

1. A multichannel spectrometer for examining naevi, having an evaluating and display device and optical means to determine and display reflectance spectra of a skin surface in an analyzable image plane while considering a white standard, characterized in that the optical means include a system of lenses (18,21,22) directed onto the skin surface and the white standard (17) and a diaphragm (19) succeeded by a diffraction grating (23) aligned with a slit (20) of the diaphragm (19), in that the diffraction grating (23) is arranged between two imaging lenses (21, 22) of the lens system (18, 21, 22) for the simultaneous production of the reflectance spectrum of the points of a determinable stripe (25) of the skin surface and of the white standard (17) in the image plane (x,y) succeeding the diffraction grating (23) and constituted by a two-dimensional detector and connected to the evaluating and display device (14).

2. A spectrometer as claimed in Claim 1, **characterized in that** the lens system (18,21,22) comprises on the optical axis (15) of the imaging lenses (21, 22) an objective lens (18) with means for sharply imaging the skin surface and the white standard (17) on the diaphragm (19).

3. A spectrometer as claimed in Claim 2, **characterized in that** the white standard (17) consists of a tablet of, for example, compressed barium sulphate secured for example, sealed, in at least the evaluated stripe (25) of the skin surface.

4. A spectrometer as claimed in Claim 3, **characterized in that** the tablet is secured beside a naevus (16) on the skin surface or on a smaller section of the naevus (16) and is displayed together with the naevus (16) by the objective lens (18).

5. A spectrometer as claimed in Claim 4, **characterized in that** the spectrometer (10) can be moved and adjusted orthogonally to the slit direction (20) and to the optical axis (15) to produce emission spectra of several stripes (25) of the skin surface to be examined.

6. A spectrometer as claimed in Claim 5, **characterized in that** the spectrometer (10) comprises a housing (11) by means of which it can be mounted infront of a low-light-level camera instead of an objective.

7. A spectrometer as claimed in Claim 6, **characterized in that the means for optically sharp imaging are constituted by** an unit for linearly moving the objective lens (18), or in that the objective lens (18) is integrated in a zoom objective with variable focus.

8. A spectrometer as claimed in Claim 7, **characterized in that** the detector (12), is associated with the evaluating and display device (14) which performs the calculation and display of the colour coordinates for each point of the evaluated stripe (25) from the standardised reflectance spectrum and tabulated standard spectral value curves which are stored, for example, in the evaluating device.

9. A spectrometer as claimed in one or more of the preceding Claims, **characterized in that** at least the stripe (25) of the skin surface and of the white standard (17) coming up for evaluation is exposed to light.

## Revendications

1. Spectromètre multicanal pour l'examen de taches pigmentaires comprenant un dispositif d'exploitation et d'affichage et des moyens optiques pour la détermination et pour la formation de l'image de spectres de réflectance d'une surface de la peau sur un plan d'image lisible, compte tenu d'un étalon de blanc, caractérisé en ce que les moyens optiques comprennent un système de lentilles (18, 21, 22) dirigé vers la surface de la peau et l'étalon de blanc (17), un écran à fente (19) et un réseau de diffraction (23) disposé en aval et aligné suivant une fente (20) de l'écran à fente (19), que le réseau de diffraction (23) est monté entre deux lentilles de formation d'image (21, 22) du système de lentilles (18, 21, 22) pour la production simultanée du spectre de réflectance des points d'une ligne exploitable (25) de la surface de la peau et de l'étalon de blanc (17) sur le plan d'image (x, y) agencé derrière le réseau de diffraction (23), connecté au dispositif d'exploitation et d'affichage (14) et constitué par un détecteur bidimensionnel.

2. Spectromètre suivant la revendication 1, caractérisé en ce que le système de lentilles (18, 21, 22) comprend sur l'axe optique (15) des lentilles de formation d'image (21, 22) une lentille objectif (18) avec des moyens pour la formation d'une image nette de la surface de la peau et de l'étalon de blanc (17) sur l'écran à fente (19).

3. Spectromètre suivant la revendication 2, caractérisé en ce que l'étalon de blanc (17) consiste en au moins une pastille, par exemple en sulfate de baryum pressé, fixée, par exemple collée, sur la ligne précisément exploitée (25) de la surface de la peau.

4. Spectromètre suivant la revendication 3, caractérisé en ce que la pastille est fixée à côté d'une tache pigmentaire (16) sur la surface de la peau ou sur une plus petite section de la tache pigmentaire (16) et son image, conjointement avec celle de la tache pigmentaire (16) est formée par la lentille objectif (18).

5. Spectromètre suivant la revendication 4, caractérisé en ce que le spectromètre (10) peut coulisser et être réglé perpendiculairement à la direction de la fente (20) et à l'axe optique (15) pour la production de spectres de réflectance de plusieurs lignes (25) de la surface de la peau qu'il faut examiner.

6. Spectromètre suivant la revendication 5, caractérisé en ce que le spectromètre (10) comprend un boîtier (11) avec lequel il peut être monté à la place d'un objectif devant un dispositif électronique à intensification de lumière résiduelle.

7. Spectromètre suivant la revendication 6, caractérisé en ce que les moyens pour la formation d'une image optiquement nette sont constitués par une unité de coulissement linéaire de la lentille objectif (18) ou la lentille objectif (18) est intégrée dans un objectif zoom à longueur focale variable.

8. Spectromètre suivant la revendication 7, caractérisé en ce qu'au détecteur (12) est adjoint le dispositif d'exploitation et d'affichage (14) qui assure le calcul et l'affichage des coordonnées chromatiques pour chaque point de la ligne (25) respectivement exploitée à partir du spectre de réflectance normalisé et des courbes de valeurs spectrales normales tabulées qui sont, par exemple, mémorisées dans l'unité d'exploitation.

9. Spectromètre suivant une ou plusieurs des revendications précédentes, caractérisé en ce qu'au moins la ligne (25) de la surface de la peau et de l'étalon de blanc (17) qui est soumise à son tour à l'exploitation est éclairée avec de la lumière.
